# EUROPEAN PATENT APPLICATION

(11) **EP 4 342 400 A1**
(43) Date of publication of application: **27.03.2024**
(21) Application number: 22197811.7
(22) Date of filing: 26.09.2022
(51) Int. Cl.: A61B 18/02

(54) **CRYOGENIC ABLATION DEVICE**

(71) Applicant: Sedivention GmbH, 82064 Strasslach (DE)
(72) Inventor: Milanovic, Gilbert, Montreal, H4C 1B9 (CA); Thibault, Benoit, Coteau-du-Lac, J0P 1B0 (CA); Mihalik, Teresa, Montreal, H3X 3P8 (CA); Charbonneau, David, Montreal, H2C 2E3 (CA); Tremblay, Andre, Oka, J0N 1E0 (CA); Delaloye, Stéphane, 8180 Bülach (CH)
(74) Representative: SSM Sandmair

(57) **Abstract**

The cryogenic ablation device of the present invention uses a first, distal balloon and a second, proximal balloon mounted on a shaft. The proximal balloon is used to position the distal balloon, and the distal balloon, which is also called cryogenic balloon, is supplied with a cooling medium to perform the cryo-ablation. The shaft is used to bring the balloons to the intended position within a patient and provides multiple lumens, for example for supplying media to the proximal balloon and the distal balloon, respectively.

## Description

### FIELD OF THE INVENTION

The present invention relates to a cryogenic ablation device comprising a cryogenic balloon for performing cryogenic ablation, in particular of the stomach, using a cooling medium supplied to the cryogenic balloon.

### TECHNICAL BACKGROUND

Obesity is the cause of many severe diseases such as diabetes, damage to joints, or heart failure. In addition to non-invasive treatments such as dieting or sporting activities, there are also effective invasive surgical and interventional treatments such as sleeve gastrectomies, gastric band operations, intragastric balloons, gastric bypasses, gastric pacemakers, Botox injections or ablating the gastric branch of the vagus nerve.

The principle of circumferential cryoablation is applied when ablating pulmonary veins in atrial fibrillation.

### BRIEF SUMMARY OF INVENTION

The present invention relates to a cryogenic ablation system for use in cryoablation, in particular cryoablation of the gastric truncs of the vagal nerve running on the gastric wall from the anterior and posterior fundus towards the pylorus.

Aspects of the present invention, examples and exemplary steps and their embodiments are disclosed in the following. Different exemplary features of the invention can be combined in accordance with the invention wherever technically expedient and feasible.

### EXEMPLARY SHORT DESCRIPTION OF THE INVENTION

In the following, a short description of the specific features of the present invention is given which shall not be understood to limit the invention only to the features or a combination of the features described in this section.

The cryogenic ablation device of the present invention uses a proximal balloon and a distal balloon mounted on a shaft. The proximal balloon is used to position the distal balloon, and the distal balloon is supplied with a cooling medium to perform the cryo-ablation. The shaft is used to bring the balloons to the intended position within a patient and provides multiple lumens, for example for supplying media to the proximal balloon and the distal balloon, respectively.

### GENERAL DESCRIPTION OF THE INVENTION

In this section, a description of the general features of the present invention is given for example by referring to possible embodiments of the invention.

The present invention relates to a cryogenic ablation device comprising a shaft having a proximal and a distal end, a first balloon located at the distal end of the shaft and a second balloon located on the shaft proximally to the first balloon. The first balloon is a cryogenic balloon, also referred to as distal balloon or cryo-balloon. The second balloon is a positioning balloon, also referred to as proximal balloon or positioning and protection balloon. The shaft can for example be a multi-lumen catheter.

In this context, the expression "at the distal end of the shaft" can mean that the shaft does not distally protrude beyond the first balloon at all or distally protrudes beyond the first balloon by a maximum length, which can be an absolute length like 5 cm, 2 cm, 1 cm, 0,5 cm, 0.2 cm or 0.1 cm or a fraction of the length of the shaft, like 5 %, 2 %, 1 %, 0.5 %, 0.2 % or 0.1 % of the length of the shaft.

The distal end of the shaft can be located within the first balloon. In this case, the first balloon can pivot about the distal end of the shaft. In another embodiment, the first balloon is completely located on the shaft, which means that both a proximal end of the first balloon and a distal end of the first balloon are connected to the shaft.

In use, the distal end of the shaft is introduced into a patient to bring the first balloon and the second balloon, both in a deflated state, to a target location at which the ablation is to be performed. For example, the distal end of the shaft is orally inserted and guided into the stomach through the esophagus. The shaft is preferably flexible.

The shaft includes at least one first outlet within the first balloon and at least one first supply lumen ending in the at least one first outlet. Through the first supply lumen and at the first outlet, a cryogenic medium, such as N₂O or CO₂, is supplied into the first balloon. The cryogenic medium inflates the first balloon such that it abuts or rests against the tissue to be treated. The cryogenic medium then subducts thermal energy from the tissue which is in contact with the first balloon.

The first outlet can act as a throttle to expand the cryogenic medium as it moves from the first supply lumen into the first balloon.

The first outlet can be referred to as cryogenic outlet and the first supply lumen can be referred to as cryogenic supply lumen.

The other end of the first supply lumen, that is the end not ending in the at least one first outlet, is connected to a cryogenic medium source, for example a cryogenic medium tank or cartridge. This connection can be direct or indirect, which means through an additional conduct located on the proximal end of the shaft.

The shaft further includes at least one second outlet within the second balloon and at least one second supply lumen ending in the at least one second outlet. Through the second outlet and the second supply lumen, a fill medium is supplied into the second balloon. The fill medium can, for example, be a liquid, like water or saltwater, or gaseous, such as air. The fill medium inflates the second balloon.

The other end of the second supply lumen, that is the end not ending in the at least one second outlet, is connected to a fill medium source, for example a syringe or a pump connected to a fill medium tank. This connection can be direct or indirect, which means through an additional conduct located on the proximal end of the shaft. The medium can for example be supplied to and removed from the second balloon through the same second lumen, which means that the second supply lumen can convey the fill medium in two opposite directions.

The second balloon has several functions. The first function is positioning the cryogenic ablation device, and therefore the first balloon, at the target location. In the case of ablation of the stomach, the second balloon prevents the shaft, and therefore the first balloon, to be pulled into the esophagus. The second function is straightening the wall of the stomach when a pulling force is applied to the shaft. This pulling force straightens the stomach in the craniocaudal, or longitudinal, direction such that the first balloon abuts the stomach wall along a closed curve. This enables a complete circumferential ablation.

Each of the first balloon and the second balloon can have a hull that is tightly connected to the shaft about the complete outer circumference of the shaft. Tightly connected means that the cryogenic medium can not escape between the shaft and the first balloon and the fill medium cannot escape between the shaft and the second balloon. The combination of the shaft and the first balloon or the second balloon, respectively, thus forms a volume that contains the cryogenic medium or the fill medium, respectively.

In one embodiment, the second balloon and the first balloon are made of a single hull which is tightly connected to the shaft at at least two positions: at the proximal side of the second balloon and at the delimitation of the second balloon and the first balloon. It can optionally further be tightly connected to the shaft at the distal side of the first balloon. However, the second balloon and the first balloon can be made of completely independent hulls.

In one embodiment, the outer shapes of the first balloon and the second balloon are rotationally symmetric about the shaft. This means that the cross-section in a plane perpendicular to the shaft of each of the balloons is a circular. In this embodiment, the cryogenic ablation device is basically rotation invariant, at least at the target location. The rotational alignment of the shaft, and thus of the balloons, at the target location is therefore irrelevant when using the device. However, it is possible to adjust the shape of the first balloon and/or the second balloon depending on the shape of the tissue to be treated.

The shape can be disk-like with parallel or slanted shoulders or donut-like, for example.

It shall be noted that the shaft is typically flexible such that its distal end can be navigated to the target location. The shaft has a shaft axis, which is defined as the center of the shaft along the proximo-distal direction of the shaft. A plane perpendicular to the shaft then means that the plane is perpendicular to the tangent to the shaft axis at the position at which the plane intersects the shaft.

In one embodiment, the diameter of the second balloon is 2.5 to 5 centimeters and the diameter of the first balloon is 1.25 to 2 times the diameter of the second balloon. In this document, all sizes and/or ratios are given for the balloons being in their inflated state with a nominal pressure unless indicated otherwise. The nominal pressure is larger than the pressure at the target location, for example by up to one atmosphere, in particular by 0.5 atmospheres.

In a cryogenic ablation device for gastric ablation, the diameter of the first balloon is for example larger than 5 centimeters, for example between 6 and 10 centimeters. The diameter of the second balloon is for example 3 to 4 centimeters.

In one embodiment, the first balloon and the second balloon are short. Here "short" means that the largest diameter of a balloon is larger than the length of the balloon, for example two, three or four times as large. The length of the balloon means the distance along the shaft at which the two ends (the proximal end and the distal end) of the balloon are tightly connected to the shaft. It could also be measured as the largest extent of the balloon in the shaft direction. The length of the second balloon and/or the first balloon is for example 25 millimeters or less.

The short property of the balloons, in particular of the first balloon, results in a smaller contact area between the balloon and the tissue to be treated. So in one implementation, the first balloon is short and the second balloon is not necessarily short.

The second balloon and/or the first balloon are for example made of a semi-compliant material. A balloon made of semi-compliant material, that is a semi-compliant balloon, only undergoes a small increase in the size with increasing inner pressure once the balloon has reached its nominal size.

In one embodiment, the distance between the first balloon and the second balloon along the shaft is 5 centimeters or less, for example 2 centimeters, 1 centimeter, 0.5 centimeters or even less. Here the distance means the distance between the distal end of the second balloon and the proximal end of the first balloon. An end of a balloon is a point at which it is tightly connected to the shaft.

The distance between the first balloon and the second balloon is typically selected depending on the desired application of the cryogenic ablation device. In the case of the ablation of the gastric wall, the height, in the craniocaudal direction, at which the tissue is to be ablated is defined by the distance between the second balloon and the first balloon if a pulling force is applied to the shaft such that the second balloon abuts the esophagus. The first balloon then has a defined distance to the cardia.

In one embodiment, the shaft further comprises a first exhaust lumen connected to the inside of the first balloon. Through the first exhaust lumen, the cryogenic medium can be removed from the first balloon, for example for deflating the first balloon or for enabling a constant stream of the cryogenic medium, thus removing cryogenic medium heated during the ablation and supplying fresh cryogenic medium. The first exhaust lumen can be connected to a pump or any other device that removes the cryogenic medium from the first balloon. This connection can be made at the proximal end of the shaft, either directly or indirectly through an additional conduct located on the proximal end of the shaft.

In one embodiment, the shaft comprises a plurality of first outlets distributed around the circumference of the shaft or at the distal end of the shaft. The plurality of cryogenic outlets are for example equally distributed around the outer circumference of the shaft. The plurality of outlets are at the distal end of the shaft for example if the shaft ends inside the first balloon.

With the plurality of first outlets, the temperature at the outer circumference of the first balloon which is in contact with the tissue can be made as uniform as possible. The shaft for example comprises 4, 6, 8, 10, 12 or 16 first outlets.

In one embodiment, the number of first supply lumens equals the number of first outlets. In this embodiment, each of the first supply lumens is associated with and ends in exactly one first outlet. By adjusting the supply of the cryogenic medium to the plurality of first supply lumens, the temperature distribution at the circumference of the first balloon which is in contact with the tissue can be controlled. The more cryogenic medium is applied to a particular first outlet, the lower the temperature at the outer circumference of the first balloon at a position facing the particular first outlet.

However, it is possible to provide less first supply lumens than first outlets. In this case, one first supply lumen can end in two or more first outlets.

In one embodiment, the first supply lumens are uniformly distributed around the inner circumference of the shaft. However, it is also possible to group two or more first supply lumens together, wherein the groups of first supply lumens are uniformly distributed around the inner circumference of the shaft.

In one embodiment, the shaft further comprises a vent lumen having an outlet to the outside of the shaft at the distal end of the shaft. The vent lumen for example connects the distal end of the shaft with the outside of the patient's body. In this embodiment, the distal end of the shaft can mean any position on the shaft, or at the distal end face of the shaft, which is more distal than the distal end of the first balloon. In the case of gastric wall ablation, the vent lumen connects the stomach with the outside of the patient to allow digestion gases to escape from or outside air to enter into the stomach. If the first balloon bursts, the cryogenic medium can escape the stomach through the vent lumen, which avoids overexpansion of the stomach or discomfort. The same applies for the fill medium if the second balloon bursts.

In one embodiment, the vent lumen and the second supply lumen are located at radially opposite sides within the shaft. This leads to a basically mirror symmetric cross-section of the shaft.

In one embodiment, the vent lumen and the second supply lumen are located within the wall of the shaft. This means that the wall of the shaft forms both the vent lumen and the second supply lumen.

In one embodiment, at least one of, and preferably each of, the at least one first supply lumen, the at least one second supply lumen and the vent lumen has a circular cross-section in a plane perpendicular to the shaft.

In one embodiment, the inside of the shaft which is not occupied by the at least one first supply lumen, the at least one second supply lumen and the vent lumen, as far as applicable, forms the first exhaust volume.

The shaft for example has a circular cross section. In one embodiment, the shaft has an outer diameter of less than 8 millimeters, for example 5 to 7 millimeters, in particular 6 millimeters.

In one embodiment, the second balloon and/or the first balloon has/have two shoulders, which are tightly connected to the shaft at a radially inner end of the respective shoulder, and a connecting section connecting the radially outer ends of the two shoulders. The shoulders and the connecting section may be formed integrally or of two or more pieces that are joined together. In one example, the balloon is continuous at the transition from a shoulder to the connecting section. This means that, in an intersection of the balloon with a plane in which the shaft axis lies, the tangents of the shoulder and of the connecting section are identical at the transition point.

In one embodiment, the shoulders, for example of the first balloon, have a truncated conical shape with an opening angle between 70 and 85 degrees, for example between 72 and 78 degrees, in particular 75 degrees.

This means that, in an intersection of the side wall with a plane parallel to the shaft axis, the angle between the shoulders is 140 to 170 degrees, for example 144 to 156 degrees, in particular 150 degrees.

The conical shoulders are for example centered about the shaft axis at the point at which the shoulders are connected to the shaft.

In one embodiment, the connecting section is a curved connection between the radial outer ends of the shoulders. The curved connection has a constant radius of 4 to 7 millimeters, for example, and in particular 5 millimeters.

The shaft may have a fitting at its proximal end or close to the proximal end. Close to the proximal end for example means that it begins less than 15 centimeters, 10 centimeters or 5 centimeters away from the proximal end. The only limitation is that the fitting is located outside the patient if the first balloon is at its target location. The fitting has at least one connector, wherein a connector is connected to one of the first supply lumen, the second supply lumen, the first exhaust lumen or the vent lumen. The connector may be standardized, such as a Luer connector.

In one embodiment, the cryogenic ablation device comprises a cryogenic medium supply conveyor, such as a tank or cartridge, connected to the first supply lumen for supplying the cryogenic medium through the first supply lumen into the first balloon. The cryogenic medium supply conveyor is for example connected to a corresponding connector of the fitting of the shaft.

In one embodiment, the cryogenic ablation device comprises a cryogenic medium exhaust conveyor, such as a pump, connected to the first exhaust lumen for removing the cryogenic medium from the first balloon through the first exhaust lumen. The cryogenic medium exhaust conveyor is for example connected to a corresponding connector of the fitting of the shaft. Instead of using a cryogenic medium exhaust conveyor, the cryogenic medium may be exhausted from the first balloon solely by the pressure inside the first balloon. The cryogenic medium exhaust conveyor may facilitate deflation of the first balloon for removal from the target location.

In one embodiment, the cryogenic ablation device comprises a fill medium conveyor, such as a syringe, connected to the second supply lumen for supplying the fill medium through the second supply lumen to the second balloon and/or for removing it therefrom. The fill medium exhaust conveyor is for example connected to a corresponding connector of the fitting of the shaft.

In one embodiment, the cryogenic ablation device comprises at least one marker. This marker can be used to locate the distal end of the shaft and/or at least one of the second balloon and the first balloon in a medical image of the patient. The marker can be radiopaque to be visible in an x-ray image or a marker that is visible in an ultrasound image.

In one implementation, the marker can be located at the distal end of the shaft. This means that it is located more distal than the first balloon. In another implementation, the marker can be attached to a balloon. The marker can for example have the shape of a circular ring centered about the shaft and may be part of or located on the hull of the balloon.

In yet another implementation, the marker is located on a part of the shaft that remains outside of the patient when the first balloon is at its target position. With such a marker, the insertion depth of the shaft can be monitored, for example before inflating the first balloon.

For example, the invention does not involve or in particular comprise or encompass an invasive step which would represent a substantial physical interference with the body requiring professional medical expertise to be carried out and entailing a substantial health risk even when carried out with the required professional care and expertise.

For example, the invention does not comprise a step of inserting the distal end of the shaft and the balloons into the patient. For this reason alone, no surgical or therapeutic activity and in particular no surgical or therapeutic step is necessitated or implied by carrying out the invention.

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1: illustrates the general structure of a cryogenic ablation device;
- Fig. 2: shows a sectional view of the device of figure 1;
- Fig. 3: shows supply and removal of a fill medium to and from a second balloon;
- Fig. 4: shows supply and removal of a cryogenic medium to and from a first balloon;
- Fig. 5: shows a sectional view of the first balloon; and
- Fig. 6: shows the shape of the first balloon.

### DESCRIPTION OF EMBODIMENTS

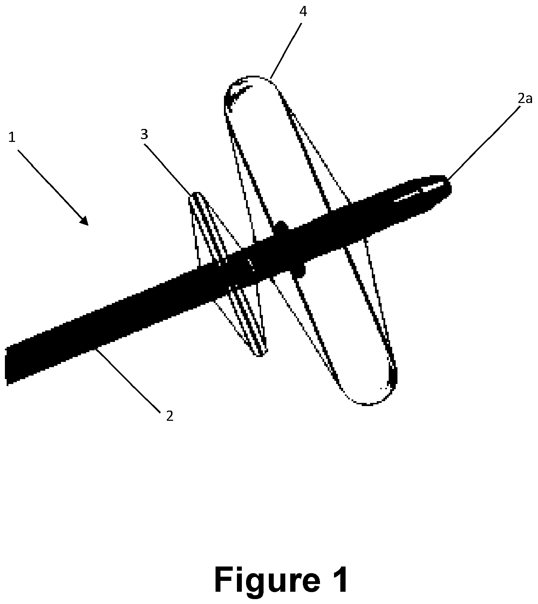

Figure 1 shows an exemplary structure of a cryogenic ablation device 1 according to the present invention. The device 1 comprises a flexible shaft 2 having a distal end 2a, a second balloon 3 and a first balloon 4. The first balloon 4 is also referred to as cryogenic balloon. The second balloon 3 and the first balloon 4 are tightly attached to the shaft 2. This means that the shaft 2 and the second balloon 3 form a second volume for a fill medium and the shaft 2 and the first balloon 4 form a first volume for a cryogenic medium.

The cryogenic ablation device according to the present embodiment is designed for a cryogenic ablation of the gastric wall. The shaft 2, the second balloon 3 and the first balloon 4 have appropriate sizes for this application.

In the present exemplary embodiment, the outer shapes of the second balloon 3 and the first balloon 4 are rotationally symmetric about the shaft 2. The first balloon 4 is closer to the distal end 2a of the shaft 2 than the second balloon 3.

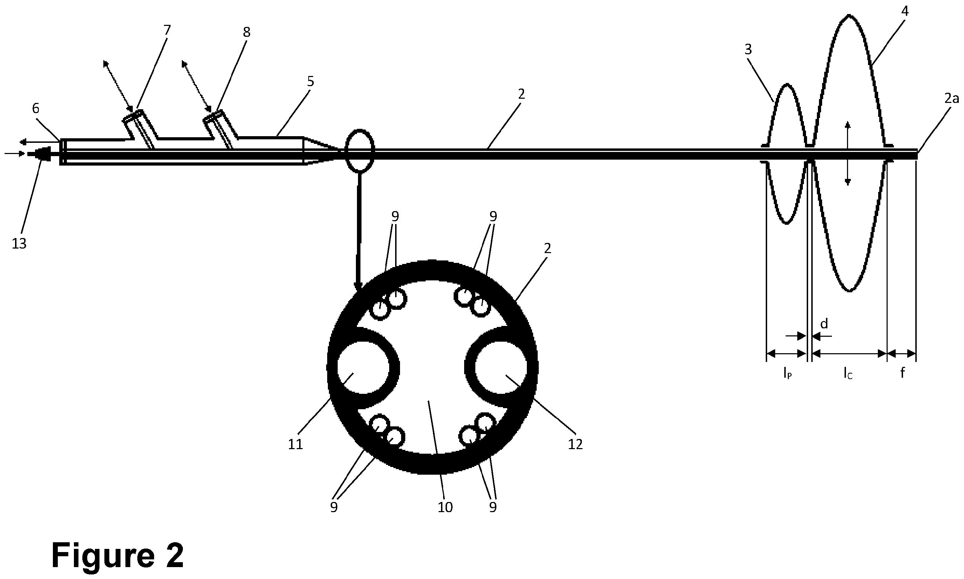

The upper part of figure 2 shows a sectional view of the cryogenic ablation device 1, while the lower part of figure 2 shows an enlarged cross-section of the shaft 2 in a plane perpendicular to the plane of the cross-section of the upper part of figure 2.

As can be seen from figure 2, the length lₚ of the second, proximal balloon 3 is smaller than the length l_{c} of first, cryogenic balloon 4. In the present embodiment, the length lₚ of the second balloon 3 is in the range of 4 to 25 millimeters, for example about 16 millimeters and the length l_{c} of the first balloon 4 is in the range of 5 to 35 millimeters, for example about 24 millimeters. In this embodiment, the term "length" means the extent of the volume enclosed by the corresponding balloon in the direction of the shaft 2. This extent ends where the respective balloon is tightly connected to the shaft 2.

In the present embodiment, the distance f between the distal end 2a of the shaft 2 and the first balloon 4 is smaller than the length l_{c} of the first balloon 4, and is for example about 1 to 10 millimeters. The distance d between the second balloon 3 and the first balloon 4 along the shaft 2 is less than one fourth of the length l_{P} of the second balloon 3, and is for example 1 to 4 millimeters. The distance d means the distance between the distal end of the volume enclosed by the second balloon 3 and the proximal end of the volume enclosed by the first balloon 4. Here an end of a volume is where the corresponding balloon is tightly connected to the shaft 2.

The cryogenic ablation device 1 further has a fitting 5 at the proximal end of the shaft 2.

As shown in the lower part of figure 2, the shaft 2 has a circular outer shape and comprises a plurality of different lumens. First supply lumens 9 supply a cryogenic medium to the first balloon 4. Second supply lumen 11 supplies a fill medium to the second balloon 3. Vent lumen 12 connects the distal end 2a of the shaft 2 with a stomach vent port 8 of the fitting 5. Through the vent lumen 12 and the stomach vent port 8, air or other gases can flow from the stomach to the outside of the patient or vice versa.

The rest of the volume inside the shaft 2 forms a first exhaust lumen 10 for removing the cryogenic medium from the first balloon 4.

As shown in the lower part of figure 2, the second supply lumen 11 and the vent lumen 12 are formed within the wall of the shaft 2.

Within the fitting 5, the second supply lumen 11 is connected to a fill medium port 7 through which a fill medium can be supplied from a fill medium reservoir (not shown), such as a syringe, to the second balloon 3 via the second supply lumen 11. On the other hand, the fill medium can be returned from the second balloon 3, for example to the fill medium reservoir, through the second supply lumen 11 and the fill medium port 7.

At the proximal end of the shaft 2, a connector 13 is connected to the first supply lumens 9 to allow flow of a cryogenic medium from a cryogenic medium reservoir (not shown) through the first supply lumens 9 into the first balloon 4. The first exhaust lumen 10 is connected to and ends at exhaust port 6. The cryogenic medium is removed from the first balloon 4 through the first exhaust lumen 10 and the exhaust port 6. This can occur through the pressure within the first balloon 4 or by an additional pump (not shown) connected to the exhaust port 6.

Initially, the second balloon 3 and the first balloon 4 are deflated, for example for introducing the distal end 2a of the shaft 2, together with the two balloons, through the esophagus into the stomach.

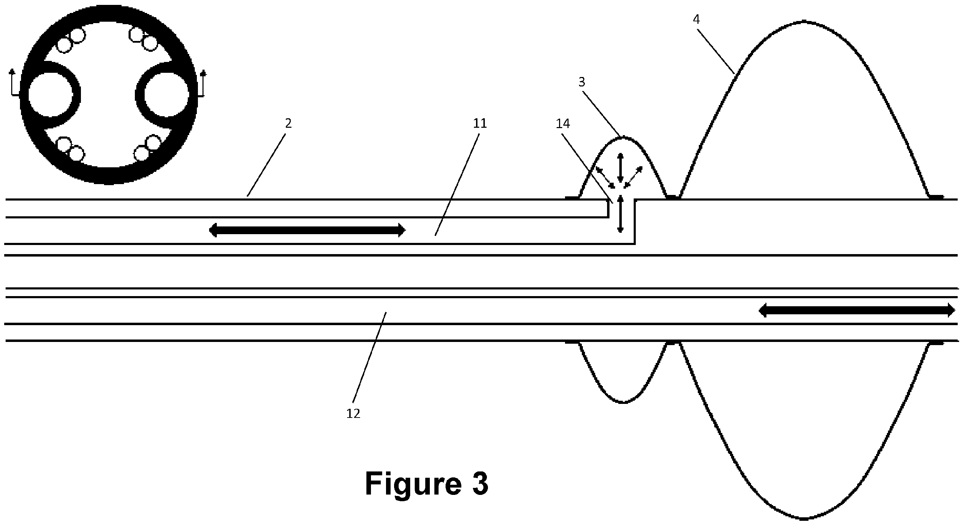

Figure 3 is a sectional view of a part of the cryogenic ablation device 1, wherein the sectional plane passes through the second supply lumen 11 and the vent lumen 12. As shown in this figure, the cryogenic ablation device 1 comprises a second supply outlet 14 within the second balloon 3. The second supply lumen 11 ends in the second supply outlet 14. The fill medium can be supplied to the second balloon 3 through the second supply lumen 11 and the second supply outlet 14 for inflating the second balloon 3. For deflating the second balloon 3, the fill medium is removed through the second supply outlet 14 and the second supply lumen 11. The movement of the fill medium is indicated by the arrows within the second supply lumen 11, the second supply outlet 14 and the second balloon 3.

For cryogenic ablation of the gastric wall, the distal end 2a as well as the first balloon 4 and the second balloon 3 are inserted into the stomach and the second balloon 3 is inflated first. Then the shaft 2 is pulled into the craniocaudal direction, which causes the second balloon 3 to be in contact with the gastric wall and to slightly stretch the stomach. Stretching the stomach has the effect that the first balloon 4 is in contact with the gastric wall along its complete circumference after inflation. The force with which the shaft 2 is pulled can for example be in the range from 0.5 kilograms to 2.5 kilograms, for example 1 kilogram or 10 Newton. The first balloon 4 can be inflated before or after pulling the shaft 2, but after inflating the second balloon 3.

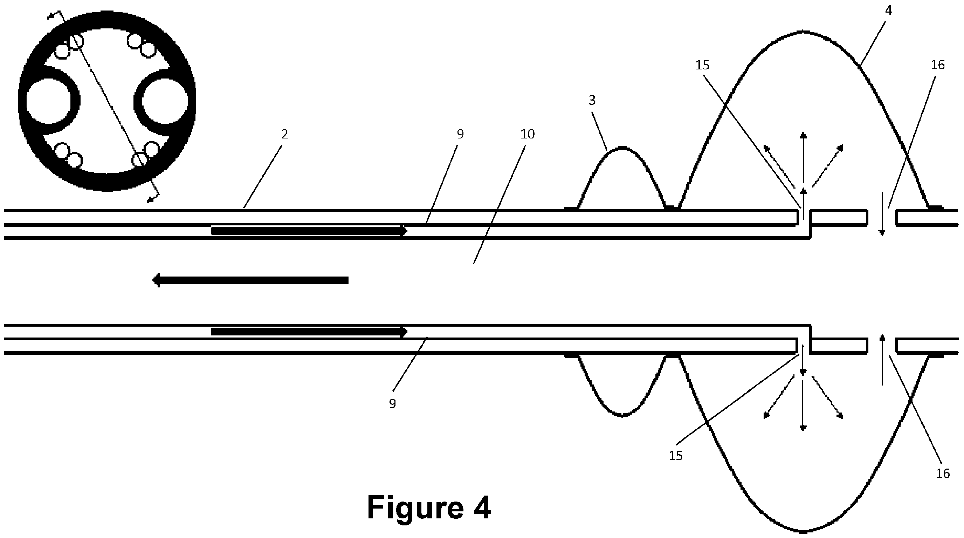

Like figure 3, figure 4 is a sectional view of a part of the cryogenic ablation device 1, but the sectional plane is slightly rotated about the axis of the shaft 2 and intersects some of the first supply lumens 9. As shown in figure 4, the shaft 2 includes a plurality of first outlets 15 in the first balloon 4. Each first outlet 15 is connected to one of the first supply lumens 9 that allows the cryogenic medium, for example N₂O or CO₂, supplied through the first supply lumens 9, to flow towards the first balloon 4 and throttle through the first outlet 15 within the first balloon 4, thus cooling its inner surface (Joule-Thomson effect). As shown by the arrows, the expanded cryogenic medium distributes within the first balloon 4 and sprays against the inner surface of the first balloon 4, thus freezing the tissue which is in contact with the first balloon 4 through thermal conduction.

The shaft 2 further comprises first exits 16 within the first balloon 4 and connected to the first exhaust lumen 10. Once the cryogenic medium has cooled the hull of the first balloon 4, it exits the first balloon 4 through the first exits 16 and the first exhaust lumen 10. This means that there is a continuous flow of the cryogenic medium for continuously cooling the inner surface of the first balloon 4.

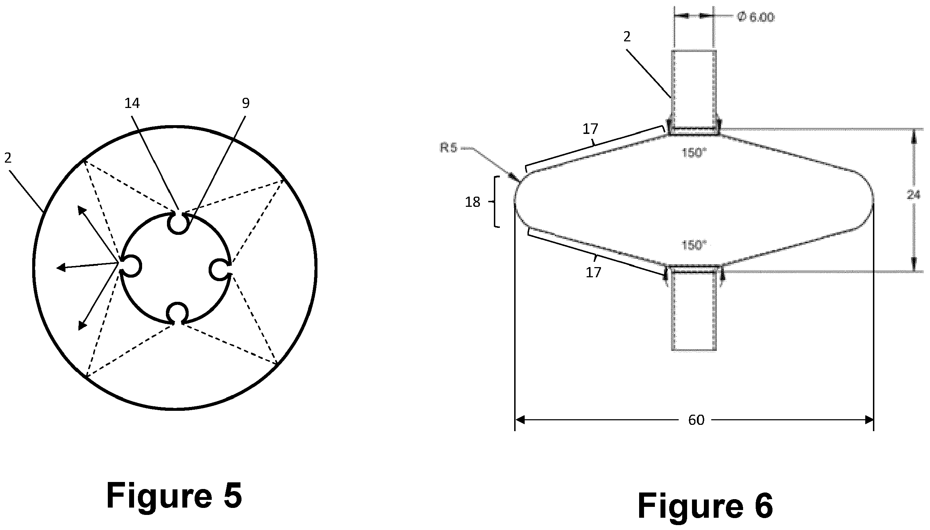

Figure 5 is a sectional view of the shaft 2 and the first balloon 4, wherein the sectional plane is perpendicular to the axis of the shaft 2. In the embodiment of the cryogenic ablation device 1 shown in this view, the shaft 2 includes four first outlets 14 and four first supply lumens 9. Only one first outlet 14 and one first supply lumen 9 is provided with a reference number in order to keep the figure simple.

At each first outlet 14, the cryogenic medium is throttled against the inner surface of the first balloon 4 with a particular opening angle as indicated by the dashed lines. The opening angle is preferably selected such that the cryogenic medium is throttled against the complete circumference of the first balloon 4 facing the first outlets 14.

The first outlets 14 are preferably equally distributed around the outer circumference of the shaft 2. They might have the shape of a slit running in the circumferential direction of the shaft 2. In this context, the shape of a slit means that the length of a first outlet 14 in the circumferential direction of the shaft 2 is larger than the width of the first outlet 14 in the axial direction of the shaft 2.

Figure 6 shows exemplary dimensions of the first balloon 4. The first balloon 4 is rotationally symmetric about the shaft 2 with a maximum diameter of 60 millimeters. It has a length of 24 millimeters. The first balloon 4 is formed of two shoulders 17 and a curved connection 18 connecting the radial outer ends of the shoulders 17. Each of the shoulders 17 resembles a truncated right circular cone which is tightly connected to the shaft 2 at its smaller diameter end. The conical shoulders 17 have an opening angle of 75 degrees, which is the angle between the shoulder and the axis of the shaft 2. This results in an angle of 150 degrees between opposite line segments forming the shoulders 17.

The curved connection 18 has a constant radius of 5 millimeters, but can have any shape as long as it closes the gap between the shoulders 17. The curved connection preferably transitions into the shoulders in a steady manner. In the present embodiment shown in figure 6, this is achieved by the curved connection 18 being a circular arc extending over 150 degrees. In general, the transition is steady if the circular arc extends over twice the opening angle of the conical shoulders 17.

The second balloon 3 may have the same structure comprising shoulders and a curved connection between their radial outer ends, but with a smaller diameter.

## Claims

1. A cryogenic ablation device (1) comprising:
- a shaft (2) having a proximal end and a distal end (2a),
- a first balloon (4) located at the distal end of the shaft (2), and
- a second balloon (3) located on the shaft (2) proximally to the first balloon (4), wherein the shaft (2) includes
- at least one first outlet (15) within the first balloon (4),
- at least one first supply lumen (9) ending in the at least one first outlet (15),
- at least one second outlet (14) within the second balloon (3), and
- at least one second supply lumen (11) ending in the at least one second outlet (14).

2. The device (1) of claim 1, wherein the outer shape of the first balloon (4) and the second balloon (3) is rotationally symmetric around the shaft (2).

3. The device (1) of claim 2, wherein the diameter of the second balloon (3) is 2.5 to 5 centimeters and the diameter of the first balloon (4) is 1.25 to 2 times the diameter of the second balloon (3).

4. The device (1) of any one of claims 1 to 3, wherein the first balloon (4) and the second balloon (3) are short.

5. The device (1) of any one of claims 1 to 4, wherein the distance between the first balloon (4) and the second balloon (3) along the shaft (2) is 5 cm or less, for example 2 cm or less.

6. The device (1) of any one of claims 1 to 5, wherein the shaft (2) further comprises a first exhaust lumen (10) connected to the inside of the frst balloon (4).

7. The device (1) of any one of claims 1 to 6, wherein the shaft (2) comprises a plurality of first outlets (15) distributed around the outer circumference of the shaft (2) or at the distal end of the shaft (2).

8. The device (1) of claim 7, wherein the number of first supply lumens (9) equals the number of first outlets (15).

9. The device (1) of claim 8, wherein the first supply lumens (9) are uniformly distributed along the inner circumference of the shaft (2).

10. The device (1) of any one of claims 1 to 9, wherein the shaft (2) further comprises a vent lumen (12) having an outlet to the outside of the shaft (2) at the distal end (2a) of the shaft (2).

11. The device (1) of claim 10, wherein the vent lumen (12) and the second supply lumen (11) are located at radially opposite sides within the shaft (2).

12. The device (1) of claim 10 or 11, wherein the vent lumen (12) and the second supply lumen (11) are located within the wall of the shaft (2).

13. The device (1) of any one of claims 1 to 12, wherein shoulders (17) of the first balloon (4) have a truncated conical shape with an opening angle between 70 and 85 degrees.

14. The device (1) of claim 13, wherein the first balloon (4) further comprises a curved connection (18) between the radial outer ends of the shoulders (17).

15. The device (1) of claim 14, wherein the curved connection (18) has a constant radius of 4 to 7 millimeters.
